(19) Europäisches Patentamt
European Patent Office
Office européen des brevets

(11) **EP 1 247 870 B1**

(12) **EUROPÄISCHE PATENTSCHRIFT**

(45) Veröffentlichungstag und Bekanntmachung des Hinweises auf die Patenterteilung:
**03.09.2008 Patentblatt 2008/36**

(51) Int Cl.:
***C12Q 1/37*** *(2006.01)*

(21) Anmeldenummer: **02007125.4**

(22) Anmeldetag: **28.03.2002**

(54) **Testsystem zur Bestimmung von in-vivo aktiven Proteasen der Hämostase in biologischen Flüssigkeiten**

Test system for the determination of proteases, active in-vivo in haemostasis, in biological fluids

Système d'essai pour la détermination des protéases, actives in-vivo dans l'hémostase, dans les fluides biologiques

(84) Benannte Vertragsstaaten:
**AT BE CH CY DE DK ES FI FR GB GR IE IT LI LU MC NL PT SE TR**

(30) Priorität: **03.04.2001 DE 10116586**

(43) Veröffentlichungstag der Anmeldung:
**09.10.2002 Patentblatt 2002/41**

(73) Patentinhaber: **Stief, Thomas, Dr.**
**35415 Pohlheim (DE)**

(72) Erfinder: **Stief, Thomas, Dr.**
**35415 Pohlheim (DE)**

(74) Vertreter: **Ackermann, Joachim**
**Postfach 11 13 26**
**60048 Frankfurt am Main (DE)**

(56) Entgegenhaltungen:
**DE-A- 19 756 773       DE-A- 19 904 674**
**US-A- 5 955 576**

• STIEF THOMAS W ET AL: "A simple screening assay for certain fibrinolysis parameters (FIPA)" THROMBOSIS RESEARCH, Bd. 97, Nr. 4, 15. Februar 2000 (2000-02-15), Seiten 231-237, XP002257502 ISSN: 0049-3848

**Beschreibung**

Die vorliegende Erfindung betrifft ein auf Peptidsubstraten basierendes globales Verfahren zur Bestimmung der in-vivo Aktivität von Proteasen der Hämostase sowie von Trypsin oder Subtilisin in biologischen Flüssigkeiten, das sich insbesondere zur Bestimmung der in-vivo Hämostaseaktivierung, insbesondere der Koagulationsaktivierung (GACA), von biologischen Flüssigkeiten einsetzen lässt oder zur Diagnose einer Pankreatitis und/oder des Schweregrades einer Pankreatitis.

Die Hämostase ist das System des Auf- und Abbaus von Thromben, d.h. die Hämostase umfasst den Vorgang der Blutgerinnung sowie den der Fibrinolyse. Normalerweise finden sich die Hämostaseenzyme im Blut größtenteils in inaktiver Form, lediglich ein sehr kleiner Teil der jeweils einzelnen Hämostaseenzyme, bei denen es sich hauptsächlich um Serinproteasen handelt, liegt in vivo in aktiver Form vor, d.h. die in vivo Aktivierung der Hämostase im Blut findet beim gesunden Organismus in geringem Umfang statt. Eine verstärkte in vivo Aktivierung der Hämostase ist jedoch von erheblicher pathophysiologischer Bedeutung in der Pathogenese vieler Erkrankungen. So finden sich bei Einsetzen einer pathologischverstärkten in vivo Hämostaseaktivierung (wie bei disseminierter intravasaler Gerinnung (DIC)) im Blut vermehrt Hämostaseenzyme in aktivierter Form. Die Bestimmung dieser in vivo aktiven Hämostaseenzyme, d.h. die Bestimmung des Grades der in vivo Hämostaseaktivierung, ist daher von großer medizinischer Bedeutung für die Diagnose von Krankheiten, die mit einer veränderten in vivo Hämostaseaktivierung verbunden sind.

Während es globale Tests zur Erfassung der Hämostase seit vielen Jahren gibt (z. B. Prothrombinzeit (PT), aktivierte partielle Thromboplastinzeit (APTT), Thrombinzeit (TT), Kher et al. (1997) Haemostasis 27(5): 211-8), existiert kein einfacher globaler Test, der die in vivo Hämostaseaktivierung mißt. Die üblichen Hämostasetests wie APTT, PT, TT erfassen nicht die in vivo Hämostaseaktivierung sondern bei diesen Tests wird eine Hämostaseaktivierung in vitro induziert, d.h. sie messen die Hämostasekapazität und nicht die in vivo Hämostaseaktivierung.

Die bislang verfügbaren Tests für die Hämostaseaktivierung lassen nur indirekte Rückschlüsse auf die in-vivo Hämostaseaktivierung zu. Mit einer Gruppe dieser Tests wird die Aktivität von Proenzymen der Hämostase (sogenannte Zymogene) ermittelt, die in der Regel erst in Proteasen umgewandelt werden müssen, wodurch lediglich die in vitro-Aktivität von Hämostaseenzymen Proben ermittelt wird. In einer anderen Gruppe dieser Tests werden mit aufwendigen Proteinzusätzen, insbesondere mit Antikörpern oder rekombinante Proteine, Aktivierungsmarker der Hämostase bestimmt. Beide Gruppen von Tests sind abhängig von einer Vielzahl von Einflüssen, die den in-vivo Zustand der Hämostaseaktivierung verfälschen und zu falschen Schlussfolgerungen führen können. So ist schon bei kurzzeitiger Lagerung von Proben mit einer Veränderung, insbesondere Amplifizierung der Aktivität an Hämostaseenzymen zu rechnen und ein Rückschluss auf den in-vivo Zustand wird praktisch unmöglich.

Im Stand der Technik beschriebene Hämostaseaktivierungstests sind Tests einerseits für D-Dimer, Thrombin-Antithrombin-Komplex, Prothrombinfragment F1+2 oder Faktor XIIa oder andererseits für lösliches Fibrin oder Faktor VIIa (Stief (2000) Thromb Haemost 84: 1120-1; MacCallum et al. (2000) Thromb Haemost 83(3): 421-6; Bos et al. (1999) Thromb Haemost 81(3): 467-8; Cardigan et al. (1998) Blood Coagul Fibrinolysis 9(4): 323-32). Diese Tests sind sehr kompliziert, beinhalten den Zusatz spezifischer Proteine, und lassen nur einen indirekten Rückschluss auf die in-vivo Aktivität der Hauptenzyme der Gerinnung, d.h. auf Thrombin (Faktor IIa) und/oder auf Faktor Xa und/oder auf andere Hämostaseproteasen, zu.

Aus der DE-A-19904674 ist ein Verfahren zur Bestimmung der Konzentration der Thrombininhibitoren bekannt. Die DE-A-19940389 beschreibt selektive Inhibitoren des Urokinase-Plasminogen Aktivators. Aus der DE-A-19756773 ist ein neues Verfahren und Diagnosemittel zur Hämostase-Diagnostik bekannt.

In Thrombosis Research Vol. 97, No. 4, pp. 231-7 (2000) ist ein Screening_Assay für bestimmte Parameter der Fibrinolyse bekannt.

Keine dieser Publikationen beschreibt die Anwesenheit der Hauptenzyme der Gerinnung (Thrombin bzw. Faktor Xa) in aktiver Form in-vivo. Die in diesen Publikationen beschriebenen Systeme nutzen jeweils die Anwesenheit von Hämostasefaktoren aus, die erst im Nachweisverfahren aktiviert werden bzw. beschreiben die Fibrinolyse und nicht die Gerinnung.

Die vorbekannten Tests sind sehr aufwendig, basieren auf dem Zusatz und/oder der Verwendung von teuren Proteinen (einerseits Antikörper oder andererseits reinen Hämostaseproteinen) und geben nur teilweise Aufschluß über die in vivo Hämostaseaktivierung. Die Durchführung der beschriebenen Verfahren ist folglich nicht sehr ökonomisch und durch diese Verfahren kann auch nicht der globale Status der in vivo Hämostaseaktivierung, insbesondere nicht der globale Gesamtstatus der in vivo Gerinnungsaktivierung, ermittelt werden, sondern nur ein Teilaspekt der Hämostaseaktivierung bestimmt werden.

Chromogene und/oder fluorogene Peptid-Substrate werden nach dem Stand der Technik eingesetzt, um die Aktivität von Gerinnungsinhibitoren wie Antithrombinen und/oder Heparinoiden im Blut oder um die Hämostase-kapazität (in vitro induzierte Hämostaseaktivierung) zu messen.

Bislang war es nicht bekannt, dass in-vivo eine so grosse Aktivität an Hämostaseenzymen in Blut vorhanden ist, so dass damit eine direkte Erfassung der Aktivität dieser Enzyme mittels chromogenen und/oder fluorogenen durch Hämostaseenzyme spaltbaren Substraten möglich wird. Die Erfindung beruht auf der Erkenntnis, dass eine geringgradige Aktivierung beim humanen Hämostasesystem physiologisch ist. Diese in-vivo Aktivierung ist jedoch sehr gering und wurde bislang nicht zum Ermitteln des Grades der Hämostaseaktivierung eingesetzt.

Neben der Bestimmung der in-vivo Hämostaseaktivierung ist als weitere Anwendungsmöglichkeit die Möglichkeit der Diagnose einer Sepsis und/oder einer Pankreatitis und/oder der Einstufung des Schweregrades einer Pankreatitis von besonderem Interesse. Insbesondere für die Einstufung des Schweregrades einer Pankreatitis ist bislang kein klinisch-chemischer Test verfügbar (Stand der Technik: Kylänpää-Bäck ML et al. JOP. Journal of the Pancreas (Online) 2002; 3: 34-48).

Bei einer Pankreatitis weist das Blut des Patienten die Serinprotease Trypsin im Blut auf. Durch die Bestimmung von in-vivo aktivem Trypsin wird es möglich, Aussagen über den Schweregrad einer Pankreatitis zu treffen.

Mit der vorliegenden Erfindung wird erstmals ein Verfahren bereitgestellt, mit dem die in-vivo Aktivität von Proteasen der Hämostase, insbesondere die einer in-vivo Gerinnungsaktivierung oder die in-vivo Aktivität von Trypsin oder Subtilisin gemessen werden kann, sowohl bei normalen Proben als auch bei Patienten.

Von besonderer Bedeutung ist dabei die rechtzeitige Erfassung hypercoagulabiler Zustände, da diese lebensbedrohlich sind bzw. werden können. Mit dem erfindungsgemäßen Testsystem ist es erstmals gewährleistet, Thrombophilie und Disseminierte Intravaskuläre Gerinnung (Verbrauchkoagulopathie) frühzeitig zu diagnostizieren.

Aufgabe der vorliegenden Erfindung ist es, ein Verfahren zur Bestimmung der in-vivo Aktivität von Proteasen der Hämostase, insbesondere von Serinproteasen, sowie der in-vivo Aktivität von Trypsin oder Subtilisin von biologischen Flüssigkeiten, insbesondere von bzw. in Blut oder Blutplasma, zur Verfügung zu stellen. Damit lässt sich direkt auf den Grad der in-vivo Hämostaseaktivierung bzw. auf den Grad einer Pankreatitis oder Sepsis schliessen.

Es wurde nun überraschenderweise gefunden, daß durch Inkubation von biologischen Flüssigkeiten mit chromogenen oder fluorogenen Substraten für Gerinnungsenzyme die in-vivo Aktivität von Proteasen, bevorzugt die in-vivo Aktivität von Serinproteasen, global erfaßt werden können.

Gegenstand der vorliegenden Erfindung ist deshalb ein Verfahren gemäß Anspruch 1 zur Bestimmung der in-vivo Aktivität von Proteasen der Hämostase sowie von Trypsin oder Subtilisin, bevorzugt der in-vivo Aktivität von Serinproteasen in biologischen Flüssigkeiten, dadurch gekennzeichnet, daß die biologische Flüssigkeit, nach der Entnahme, mit mindestens einem chromogenen oder fluorogenen Substrat, vorzugsweise Peptidsubstrat, für mindestens eine Protease der Hämostase sowie von Trypsin oder Subtilisin, bevorzugt eine Serinprotease, insbesondere für mindestens ein Hämostaseenzym oder für Trypsin und/oder für Subtilisin inkubiert wird und vor der Zugabe des chromogenen Substrates mindestens ein Antikoagulans aus der Gruppe EDTA, EGTA, Arginin, Guanidinium und Singlet-Oxygen Erzeuger hinzugegeben wird.

Weiterhin wird ein Testsystem zur Bestimmung der in-vivo Aktivität von Proteasen der Hämostase sowie von in-vivo aktivem Trypsin oder Subtilisin, vorzugsweise von Serinproteasen in biologischen Flüssigkeiten beschrieben, dadurch gekennzeichnet, dass das Testsystem mindestens ein chromogenes oder fluorogenes Substrat für mindestens eine Serinprotease, insbesondere für mindestens ein Hämostaseenzym oder für Trypsin und/oder für Subtilisin umfaßt.

Darüber hinaus wird die Verwendung dieses Testsystems beschrieben, insbesondere die Verwendung von chromogenen oder fluorogenen Substraten für mindestens ein Hämostaseenzym zur Bestimmung der in vivo Hämostaseaktivierung in biologischen Flüssigkeiten oder die Verwendung von chromogenen oder fluorogenen Substraten für Trypsin und/oder für Subtilisin zur Bestimmungder in-vivo Aktivität von Trypsin und/oder von Subtilisin in biologischen Flüssigkeiten.

Ferner wird ein Diagnostikum zur Bestimmung der in-vivo Hämostaseaktivierung oder zur Bestimmung der in-vivo Aktivität von Trypsin und/oder Subtilisin in biologischen Flüssigkeiten offenbart, enthaltend mindestens ein chromogenes oder fluorogenes Substrat für mindestens ein Hämostaseenzym und/oder enthaltend mindestens ein chromogenes oder fluorogenes Substrat für Trypsin und/oder für Subtilisin und gegebenenfalls weitere Hilfs- und Zusatzstoffe.

Als biologische Flüssigkeiten im Sinne der vorliegenden Erfindung sind ganz allgemein Körperflüssigkeiten aus Körperzellen abgeleitete Flüssigkeiten zu verstehen, wie Zellkulturen, die beispielsweise von Endothelzellen abgeleitet sind, Kompartmentflüssigkeiten und bevorzugt Blut und/oder Plasma, insbesondere EDTA-Blut, EDTA-Plasma, EDTA/Arginin-Blut oder EDTA/Arginin-Plasma.

Probenmaterial für die Bestimmung der Hämostaseaktivierung ist nach dem Stand der Technik Citratblut und/oder Citratplasma. Mit anderen Antikoagulantien versehene Proben, wie EDTA-Blut und/oder EDTA-Plasma bzw. EDTA/Arginin-Blut und/oder EDTA/Arginin-Plasma wurden für diesen Zweck bislang nicht eingesetzt.

Das erfindungsgemäße Verfahren wird mit Blut bzw. Plasma durchgeführt, das mit Antikoagulantien versetzt worden ist, wie mit Singlet Oxygen Erzeugern wie Chloramine, insbesondere ChloraminT® (N-Chloro-p-toluenesulfonamide), EDTA, EGTA, Arginin und/oder Guanidin und gegebenenfalls zusätzlich Heparin.

Es zeigte sich überraschenderweise, dass EDTA-Blut und EDTA-Plasma bzw. EDTA/Arginin-Blut und EDTA/Arginin-Plasma besonders gut geeignet sind, um die in vivo-Hämostaseaktivierung bzw. die in-vivo Aktivität von Trypsin und/oder Subtilisin zu bestimmen. Anstelle von EDTA bzw. Arginin können auch Guanidin bzw. Chloramin T® oder eine Kombination dieser Substanzen mit oder ohne EDTA eingesetzt werden.

Bevorzugt werden EDTA und/oder Arginin und/oder Guanidin bzw. ChloraminT® bereits bei der Blutentnahme zugesetzt.

Weiterhin wird ein Verfahren zur Diagnostik der in-vivo Hämostaseaktivierung oder der in-vivo Aktivität von Trypsin und/oder Subtilisin in einer biologischen Flüssigkeit beschrieben, wobei EDTA-Blut oder EDTA-Plasma, Arginin-Blut oder Arginin-Plasma oder EDTA/Arginin-Blut oder EDTA/Arginin-Plasma als biologische Flüssigkeit verwendet wird.

Das erfindungsgmäße Verfahren zur Bestimmung der in vivo Hämostaseaktivierung oder der in-vivo Aktivität von Trypsin und/oder Subtilisin zeichnet sich dadurch aus, dass es sich um ein sehr einfach durchführbares, präzises und sehr preiswertes Verfahren zur Bestimmung der in vivo Hämostaseaktivierung oder der in-vivo Aktivität von Trypsin und/oder Subtilisin handelt, mit dem Proben einer großen Anzahl von Patients in sehr kurzer Zeit untersucht werden können.

Mit dem erfindungsgemäßen Verfahren ist es ohne weiteres möglich, VK-Werte von weniger als 5% zu erzielen, wohingegen die vorstehend beschriebenen bekannten Tests für aktive Hämostaseenzyme lediglich VK-Werte von mehr als 10% liefern.

Bei den Größen der Hämostaseaktivierung bzw. von aktivem Trypsin und/oder Subtilisin, die erfindungsgemäß bestimmt werden können, handelt es sich insbesondere um die unkorrigierte Koagulationsaktivierung (uGACA) und um die korrigierte Koagulationsaktivierung (GACA), wobei GACA für "global assay of in-vivo coagulation activation" steht.

Ausgehend vom uGACA-Wert kann vorzugsweise die um die Kontaktphasenkapazität (CPC) korrigierte Koagulationsaktivierung (GACA) bestimmt werden.

Erfindungsgemäß wird das Verfahren zur Ermittlung des GACA-Wertes bevorzugt so durchgeführt, dass die biologische Flüssigkeit nach der Entnahme mit einem Puffer, gegebenenfalls Antikoagulantien und weiteren Zusatzstoffen, und einem chromogenen oder fluorogenen Substrat umgesetzt wird und anschließend Inkubation der so erhaltenen Mischung erfolgt.

Es hat sich gezeigt, dass das erfindungsgemäße Verfahren in der Regel bei finalen Substratkonzentrationen ≤ 2 mmol/l (entsprechend ≤ 2 mM) und/oder bei Substratmengen von ≤ 3 nmol/$\mu$l Probe) durchgeführt werden sollte. Beim Einsatz größerer Konzentrationen und/oder Mengen an Substrat kann es zu einer ungewünschten Aktivierung von Proenzymen der Hämostase kommen. Die untere Grenze der Substratkonzentration wird von der gewünschten Empfindlichkeit des Tests bestimmt. Die im Einzelfall erforderliche Konzentration und/oder Menge kann vom Fachmann anhand des in den Beispielen gegebenen Schemas bestimmt werden.

Bevorzugt setzt man finale Substratkonzentrationen von 0,05 bis 1 mM und/oder Substratmengen von ≤ 1,5 nmol/$\mu$l Probe, insbesondere von 0,05 bis 0,8 mM und ganz besonders bevorzugt von 0,5 bis 0,6 mM ein.

Im Bereich von < 1 mM, insbesondere von 0,5-0,6 mM entspricht die Bestimmung von uGACA näherungsweise dem Gesamt-GACA Wert, während unspezifische Proteasen erst bei höheren Substratkonzentrationen aktiv sind. Nur bei Verwendung von Substratkonzentrationen ≥ 1 mM empfiehlt es sich in der Regel, den erhaltenen uGACA durch die CPC zu korrigieren.

Der Anstieg der Absorption, Fluoreszenz und/oder Extinktion während der Inkubationsdauer der Probe kann mit einem Spektrometer und/oder Spektroskop bestimmt werden. Anstelle davon kann auch ein Anfangs- und Endzustand der Absorption, Fluoreszenz und/oder Extinktion ermittelt werden.

Bevorzugt erfolgt Bestimmung der Fluoreszenz, Extinktion und/oder Absorption bei einer definierten, für den Chromophor des chromogenen oder fluorogenen Substrates charakteristischen Wellenlänge, insbesondere bei einer Wellen-länge, bei der es bei Spaltung des Substrats durch das Gerinnungsenzym zu einer signifikanten, d.h. spektroskopisch und/oder spektrometrisch messbaren Änderung der Fluoreszenz, Extinktion und/oder Absorption kommt. Bei den so ermittelten Werten handelt es sich um einen Absolutwert für die Koagulations-aktivierung (uGACA).

Die Inkubation erfolgt erfindungsgemäß bevorzugt zwischen 0,5 und 180 Minuten bei einer Temperatur zwischen 30 und 50 °C, besonders bevorzugt bei einer Temperatur zwischen 37 und 45°C, ganz besonders bevorzugt bei 37°C oder zwischen 40 und 45°C. Erfolgt die Inkubation bei 37°C, so erfolgt diese bevorzugt für 0,5 - 120 Minuten, bei Inkubation zwischen 40 und 45°C erfolgt diese bevorzugt für 0,5 bis 60 Minuten.

Zur Ermittlung des prozentualen GACA-Wertes werden die ermittelten Absorptions/Fluoreszenz-Werte der Proben mit dem 100 % gesetzten Wert eines Normalkollektivs verglichen. Hierzu werden die Werte der Koagulationsaktivierung eines Standards oder von Proben von gesunden Probanden ermittelt und daraus der Mittelwert (= 100 %) gebildet. Das für die Patienten-probe erhaltene Ergebnis wird in Prozent des 100 % Normalwert-Standards (wie in Standardhumanplasma und/oder Kontrollplasma N®, DadeBehring) ausgedrückt.

Da insbesondere auch Proenzyme der Kontaktphase der Hämostase die eingesetzten chromogenen Substrate amidolytisch spalten, sollte der erhaltene unkorrigierte GACA-Wert gegebenenfalls korrigiert werden durch die Kontaktphasenkapazität (CPC) der Probe. Diese kann vorzugsweise in einem 1-step oder in einem 2-step- Verfahren bestimmt werden (vergl. Beispiele). Zur Ermittlung der 1-step-CPC kann die biologische Flüssigkeit mit einem Puffer, gegebenenfalls Antikoagulantien und weiteren Zusatzstoffen, einem chromogenen oder fluorogenen Substrat und einem Kontaktphasenaktivator umgesetzt werden. Anschließend erfolgt Inkubation der so erhaltenen Mischung, bevorzugt bei einer Temperatur zwischen 15 und 37°C, für bevorzugt nicht länger als 30 Minuten, besonders bevorzugt zwischen 0,1 und 10 Minuten, insbesondere für 0,2 bis 2 Minuten (bei 37°C).

Bestimmung der Absorption, Fluoreszenz und/oder Extinktion kann hierbei wie zuvor für das Verfahren zur Ermittlung des uGACA-Wertes beschrieben erfolgen. Werte für die Kontaktphasenkapazität von gesunden Probanden werden ermittelt, anschließend wird ein Mittelwert (100 %) gebildet. Der erhaltene Wert für die Patientenproben wird in Prozent des 100 % Wertes für die gesunden Probanden = 100 % Normalwert-Standard (wie in Standardhumanplasma und/oder Kontrollplasma N®) wiedergegeben.

Als Kontaktphasenaktivator wird erfindungsgemäß vorzugsweise Kaolin, Ellagsäure oder vorzugsweise $SiO_2$ verwendet.

Kaolin wird vorzugsweise in einer Test-Konzentration zwischen 0,1 und 4 g/l eingesetzt, besonders bevorzugt in einer Test-Konzentration zwischen 0,2 und 2 g/l, insbesondere mit 0,8 g/l.

Ellagsäure wird vorzugsweise in einer Konzentration zwischen 0,01 und 0,3 g/l eingesetzt, besonders bevorzugt zwischen 0,02 und 0,2 g/l, insbesondere mit 0,075 g/l.

Als $SiO_2$ Reagenz kann Pathromtin SL® (Dade Behring, Marburg, Deutschland) verwendet werden, 1:2 bis 1:16 fach im Test verdünnt, besonders bevorzugt 1:3 bis 1:8 fach im Test verdünnt, insbesondere 1:4 fach verdünnt.

Überraschenderweise wurde gefunden, dass die zusätzliche Zugabe des Kontaktphasenaktivators unterbleiben kann, wenn zur Bestimmung der CPC ein chromogenes Substrat verwendet wird, dessen Endgruppe als Kontaktphasenaktivator fungiert. Beispiele für solche Substrate sind methoxysulfonierte oder acetylierte Substrate für Hämostaseenzyme, wie beispielsweise $CH_3SO_2$-D-CHG-Gly-Arg-pNA·AcOH, Pefachrome®FIXa (Pefa-3107); Pentapharm, Basel, Schweiz).

Die Bestimmung des GACA-Wertes und der CPC erfolgt erfindungsgemäß bevorzugt durch Aufnahme eines Absorptions-, Fluoreszenz- und/oder Extinktionswertes bei einer bestimmten, für den Chromophor und/oder Fluorophor des chromogenen und/oder fluorogenen Substrats charakteristischen Wellenlänge.

Das Verfahren umfasst somit erfindungsgemäß besonders bevorzugt die im folgenden aufgezählten Verfahrensschritte:

a) Bestimmung der Hämostaseaktivierung für Proben von Patienten durch Zugabe eines chromogenen und/oder fluorogenen Substrates, zu jeder Probe bzw. zu einer Standardprobe, spektroskopische und/oder spektrometrische Bestimmung der Änderung der Absorption, Fluoreszenz und/oder Extinktion der Probe bei mindestens einer definierten Wellenlänge, Angabe des uGACA-Wertes, indem die Patienten-Proben mit einer bekannten Standard-Probe (Plasma oder Reinenzym (beispielsweise Faktor Xa oder Thrombin)) verglichen werden und in Prozent des Mittelwertes der gesunden Probanden dargestellt werden,

b) Bestimmung der CPC in analoger Weise wie die Bestimmung des uGACA-Wertes, nur dass zu den Proben zusätzlich ein Kontaktphasenaktivator hinzugegeben wird, und

c) Bestimmung des um die CPC korrigierten GACA-Wertes, indem der Wert für die CPC vom uGACA-Wert subtrahiert und anschließend 100 % addiert wird.

Im Bereich von < 1 mM, insbesondere von 0,05 - 0,8 mM, besonders bevorzugt 0,5 - 0,6 mM entspricht die Bestimmung von uGACA näherungsweise dem Gesamt-GACA Wert, während unspezifische Proteasen in der Regel erst bei höheren Substratkonzentrationen aktiv sind. Nur bei Substrat-Konzentrationen ≥ 1 mM ist es in der Regel empfehlenswert, den erhaltenen uGACA-Wert durch die CPC zu korrigieren.

Bevorzugt setzt man ein in EDTA und/oder in Arginin gelöstes Substrat ein, so dass eine finale Testkonzentration an EDTA von mindestens 2,1 g/l und eine von Arginin (basischen pH-Wertes) von mindestens 250 mmol/l resultiert.

Bei der Probe handelt es sich hierbei um eine biologische Flüssigkeit, bei der definierten Wellenlänge handelt es sich um eine für den Chromophor des chromogenen oder fluorogenen Substrates charakteristischen Wellenlänge.

Bei der biologischen Flüssigkeit handelt es sich erfindungsgemäß bevorzugt um Blut oder Blutplasma, besonders bevorzugt um EDTA-Blut oder EDTA-Plasma, Arginin-Blut oder Arginin-Plasma oder EDTA/Arginin-Blut oder EDTA/Arginin-Plasma.

Erfindungsgemäß wird die biologische Flüssigkeit zur Durchführung des Tests bevorzugt mit einem Puffer umgesetzt, insbesondere mit Phosphate Buffered Saline (PBS), $HCO_3^-$, Arginin, oder Tris bei pH 7-11, vorzugsweise pH 7-9, insbesondere pH 7,4 für PBS oder pH 8,7 für Arginin. Zusammen mit dem Puffer können erfindungsgemäß auch andere Antikoagulantien verwendet werden, um die in vitro erfolgende (d.h. artifizielle) Hämostaseaktivierung in der biologischen Flüssigkeit während der GACA-Testinkubation zu verhindern, wie beispielsweise Ethylene Diamine Tetraacetic Acid (EDTA), Ethylene Glycol-bis(ß-aminoethyl Ether)N,N,N',N'-Tetraacetic Acid (EGTA), Arginin, Guanidiniumsalze, oder Singlet Oxygen Erzeuger, insbesondere vom Typ der Chloramine.

Während beispielsweise bei EDTA der Zusatz weiterer Antikoagulantien nicht unbedingt erforderlich ist, ist die relativ milde Calcium-Komplexierung durch Citrat in der Regel nicht ausreichend, um eine zusätzliche in vitro Hämostaseaktivierung des Blutplasmas im Test zu verhindern, so dass hier erfindungsgemäß weitere Antikoagulantien hinzugegeben werden. Erfindungsgemäß wird so beispielsweise bevorzugt EDTA zur Stabilisierung der Blutprobe in einer Konzentration von 0,8 bis 2,4 mg/ml, insbesondere 1,6 mg/ml (4,4 mmol/l Blut entsprechend ca. 8 mmol/l Plasma) verwendet. Die Untersuchung von Citratblut erfordert den Zusatz eines weiteren Antikoagulans im Test: bei Verwendung von Citratplasma kann als weiteres Antikoagulans erfindungsgemäß beispielsweise EDTA, bevorzugt in einer Testkonzentration zwischen 2-20 mmol/l, insbesondere bei 5-10 mmol/l, und/oder Arginin, bevorzugt bei einer Testkonzentration zwischen 50-500 mmol/l, insbesondere bei 80-300 mmol/l, besonders bevorzugt 250 mmol/l, verwendet werden.

Bei den chromogenen und/oder fluorogenen Substraten für Proteasen, insbesondere für Hämostaseenzyme oder Trypsin und/oder Subtilisin handelt es sich erfindungsgemäß entsprechend um Peptide mit einer Aminosäuresequenz, die durch Hämostaseenzyme, besonders bevorzugt von Faktor Xa und/oder Thrombin und/oder durch andere Hämostaseserinproteasen, bzw. durch Trypsin und/oder durch Subtilisin amidolytisch gespalten werden kann, und die einen Chromophor tragen, wobei es bei der amidolytisch Spaltung der Substrate durch das Hämostaseenzym bzw. durch Trypsin und/oder durch Subtilisin zu einer Änderung im Absorptions- und/oder Fluoreszenzspektrum des Chromophors kommt, das spektroskopisch und/oder spektrometrisch erfasst werden kann.

Bevorzugt umfassen die Substrate 2 bis 10, besonders bevorzugt 2, 3, 4 oder 5 Aminosäuren. Bei den Aminosäuren kann es sich auch um nicht-proteinogene oder natürlicherweise nicht vorkommende Aminosäurederivate oder andere organische Verbindungen handeln. Das Substrat kann ferner modifiziert sein, etwa durch Anknüpfung von mindestens einer Schutzgruppe, mindestens einer markierenden Gruppe und/oder mindestens einer organischen Verbindung. Die chromogene und/oder fluorogene Gruppe ist bevorzugt an die carboxy-terminale Aminosäure des Substrats kovalent geknüpft.

Als chromogene Substrate eignen sich erfindungsgemäß beispielsweise Substrate für Hämostaseenzyme, die para-Nitroanilin (pNA) als Chromophor tragen, insbesondere Thrombin und/oder Faktor Xa- Substrate, beispielsweise handelt es sich um die folgenden Substrate:

HD-CHG-Ala-Arg-pNA · 2AcOH, Pefachrome®TH (Pefa-5114), Pentapharm, Basel, Schweiz;
HD-CHA-Ala-Arg-pNA, Instrumentation Laboratory (IL), Lexincton, USA;
HD-Phe-Pip-Arg-pNA · HCl (HD-Phenylalanyl-L-pipecolyl-L-arginine-para nitroaniline; S-2238®), Chromogenix, Mölndal, Schweden;
HD-HHT-Gly-Arg-pNA; HHT = Cyclohexyltyrosin; CHG = Cyclohexylglycin; CHA = β-Cyclohexylalanin; pNA = para-Nitroanilid; Pefachrome®FXIIa (Pefa-5963), Pentapharm
N-α-Z-D-Arg-Gly-Arg-pNA·2HCl (S-2765®), Chromogenix, Mölndal, Schweden;
Bz-Ile-Glu(γ-OR)Gly-Arg-pNA·HCl (S-2222®), Chromogenix, Mölndal, Schweden;
HD-Ile-Pro-Arg-pNA · HCl (S-2288®), Chromogenix, Mölndal, Schweden;
-CO-lle-Glu-(γ-OR)-Gly-Arg-pNA (FXa, Trypsin; S-2222 ®), Chromogenix
HD-Val-Leu-Arg-pNA (Kallikrein; S-2266®), Chromogenix
D-Pro-Phe-Arg-pNA (Kallikrein; S-2302®), Chromogenix.

Beispiele für Subtilisinsubstrate, insbesondere zur Diagnose einer Sepsis, sind Z-Gly-Gly-Leu-pNA, Z-Ala-Ala-Leu-pNA, Suc-Ala-Ala-Ala-pNA, Suc-Ala-Ala-Phe-pNA, und insbesondere Suc-Phe-Ala-Ala-Phe-pNA (Bachem, Heidelberg, Deutschland).

Trypsin als eine Breitsprekrum-Serinprotease spaltet insbesondere chromogene Substrate mit endständiger basischer Aminosäure-(insbesondere Arginin oder Lysin)-Chromophor Gruppe.

Für die Bestimmung der CPC lassen sich darüber hinaus Substrate einsetzen, die insbesondere durch Pro-Enzyme der Kontaktphase gespalten werden; dabei handelt es sich beispielsweise um

$CH_3SO_2$-D-CHA-Abu-Arg-pNA
Pefa IXa ($CH_3SO_2$-D-CHG-Gly-Arg-pNA)
Pefa VIIa ($CH_3SO_2$-D-CHA-But-Arg-pNA)
Pefa Xa ($CH_3OCO$-D-CHA-Gly-Arg-pNA)

Diese Substrate zeichen sich dadurch aus, dass sie selbst in Anwesenheit von Arginin (selbst in Konzentrationen > 100 mM) durch die Kontaktphasenenzyme gespalten werden.

Erfindungsgemäß wird das Verfahren beispielsweise so durchgeführt, daß zur Bestimmung der uGACA 100 μl EDTA-Plasma mit 50 μl 3 mmol/l Peptid-Substrat für 0,5-120 Minuten bei 37°C oder für die Hälfte der Zeit bei 40-45°C inkubiert werden. Gemessen wird der Anstieg der Extinktion bei 405 nm. Das Messergebnis [A] wird verglichen mit einem bekannten Standard (beispielsweise ein 100 % normales Standardhumanplasma oder Reinenzym (6 mIU/ml Thrombin oder 45 pkat/ml F Xa)) und in % ausgedrückt. Der normale uGACA-Wert ist 100 ± 50 % (Mittelwert ± 2 Standardabweichungen eines Normalkollektivs).

Zur Bestimmung der Kontaktphasenkapazität werden dann 100 μl Probe mit 50 μl chromogenem Substrat und 50 μl eines Kontaktaktivators (z.B. Kaolin 2,4 g/l (Pathromtin®, Dade Behring, Marburg, Deutschland), Ellagsäure 0,3 g/l (Neothromtin®, Dade Behring) oder $SiO_2$ (Pathromtin SL®, Dade Behring) für 4 min bei Raumtemperatur inkubiert. Auch hier wird das Ergebnis [B] mit dem eines 100 % normalen Standardhumanplasmas verglichen und in Prozent ausgedrückt.

Die Korrekturformel zur Berechnung des spezifischen GACA-Wertes ist demzufolge:

$$GACA = A - B + 100 \%$$

In einer besonders bevorzugten Ausführungsform des erfindungsgemäßen Verfahrens wird eine Endkonzentration an Substrat von weniger als 1 mmol/l eingesetzt. Bei Substratkonzentration unter 0,8 mmol/l entspricht der uGACA-Wert in der Regel dem GACA-Wert. Die Bestimmung der CPC kann somit ausgelassen werden. Daher ist ein GACA-Ansatz von besonderer Bedeutung, bei dem die Incubation von 100 μl Probe (z. B. Plasma) mit 50 μl 1,7 mmol/l Peptidsubstrat (wie beispielsweise ein Thrombinsubstrat, insbesondere CHG-Ala-Arg-pNA) in 750 mmol/l Arginin, pH 8,7, 6,4 g/l EDTA vorgenommen wird.

Der normale GACA-Wert ist 100 ± 50 % (Mittelwert ± 2 Standardabweichungen eines Normalkollektivs).

Die Erfindung soll durch folgende Beispiele erläutert werden, ohne daß diese den Erfindungsgedanken einschränken sollen:

Beispiel 1: Beispiel GACA und Beispiel CPC

Zur Durchführung des Standard GACA wird 100 μl Probe (vorzugsweise EDTA-Plasma) von a) n = 10 normalen Spendern und b) n = 35 Patienten mit 50 μl 3 mmol/l chromogenem Substrat HD-CHG-Ala-Arg-pNA in phosphate buffered saline (PBS) pH 7.4, vorzugsweise enthaltend 15-30 mmol/l EDTA, insbesondere 20 mmol/l EDTA, in einer Mikrotiterplatte gemischt und die Ausgangsextinktion bei 405 nm mittels eines Mikrotiterplattenphotometers (Milenia, DPC, Los Angeles, USA) bestimmt. Nach einer Inkubation von 90 min bei 37°C wird die Extinktion bei 405 nm erneut bestimmt und die Differenz zur Ausgangsextinktion berechnet.

Ergebnis: Der GACA Normalbereich (Mittelwert ± 2 Standardabweichungen (σ), berechnet aus Normalspendern) beträgt 100 ± 50 %, d.h. die normale uGACA-Aktivität ist 254 ± 116 mA/90 min (37°C). Das Patientenkollektiv hatte einen mittleren uGACA-Wert von 471 ± 154 mA/90 min (37°C). Dieser uGACA-Wert wurde in % Normalplasma umgerechnet [A], der ermittelte Wert betrug also 471/254 =185 %.

Zusätzlich wurde die Kontaktphasenkapazität (CPC) der eingesetzten Plasmen gemessen, um den erhaltenen uGACA-Wert zu korrigieren, d.h. einen kontaktphasenunabhängigen GACA Wert zu erhalten. Hierzu wurden 100 μl Probe mit 50 μl 3 mmol/l HD-CHG-Ala-Arg-pNA in PBS, pH 7.4, 20 mmol/l EDTA und 50 μl Pathromtin SL® ($SiO_2$; Dade Behring) 5 min bei RT in einer Mikrotiterplatte inkubiert. Daraus resultierte eine normale CPC von 1319 ± 439 mA (= 100 ± 33 %)/5 min (RT) (das Patientenkollektiv hatte eine CPC von 1339 ± 442 mA/5 min (RT)). Die CPC der einzelnen Patienten wurde umgerechnet in % Aktivität verglichen mit dem 100 % Standard-Normalplasma. Die so erhaltene Aktivität [B= CPC] wird von der Aktivität [A= uGACA] abgezogen und 100 % wird dazusummiert, was zum kontaktphasenkorrigierten GACA führt:

$$GACA = A - B + 100\%$$

Beispiel 2: Optimierung der Testkonzentration an Kontaktphasenaktivator im CPC

Zur Optimierung des Kontaktphasenaktivators im CPC wurde 100 μl

a) Neothromtin (0,6 g/l Ellagsäure (3 ml Fläschchen Dade Behring in 1,5 ml $H_2O$ gelöst))
b) Pathromtin (5 g/l Kaolin, Dade Behring)
c) Pathromtin SL ($SiO_2$, Dade Behring)

in 1:1, 1:2, 1:4, 1:8, 1:16, 1:32, 1:64, 1:128 Verdünnung mit $H_2O$ oder zur Kontrolle 100 μl $H_2O$ mit 100 μl 1,5 mmol/l HD-CHG-Ala-Arg-pNA in PBS und mit 100 μl Normalplasma (Citrat) für 4 min (RT) inkubiert. Der Extinktions-anstieg (405 nm)/ 4 min wurde bestimmt.

Ergebnis: Optimale Proteasenaktivität findet sich bei einer Testkonzentration von 0,8 g/l (0,2-2 g/l) Kaolin (delta A=2138 mA/4 min; z.B. Pathromtin Dade Behring Marburg), 0,05 g/l (0,02-0,2 g/l) Ellagsäure (delta A=1443 mA/4 min) und 17 % (8,5 - 50 %) Testkonzentration an $SiO_2$ wie in Pathromtin SL® (delta A=1375 mA/4 min).

Beispiele 3 - 19

In den nachfolgenden Beispielen wird ein Global Assay of Coagulation Activation = GACA beschrieben

Testschema GACA:

| 100 µl | Probe |
| 50 µl | 1.7 mM HD-CHG-Ala-Arg-pNA, |
| | 6.4 g/l EDTA, 750 mM Arginin, pH 8.7 |

Δ A 405 nm/t (37°C)

Testvolumina gegebenenfalls gleichsinnig veränderbar (d.h. z.B. 200 µl Probe + 100 µl Substrat)

Probe =   Plasma oder Blut, insbesondere

♦ Arginin-stabilisiertes EDTA-Plasma (2,6 ml EDTA-Blut + 300 µl 1,5 M Arginin)

♦ EDTA-Plasma (nicht älter als 2 h)

♦ Citrat-Plasma (nicht älter als 2 h)

♦ gegebenenfalls Heparin-Plasma

Desweiteren wird die Bestimmung der Contact Phase Capacity = CPC beschrieben. Testschema 1-step-CPC:

| 100 µl | Probe |
| 50 µl | 1,7 mM HD-CHG-Ala-Arg-pNA, |
| | 6,4 g/l EDTA, pH 7,4 |
| 50 µl | Pathromtin SL® |

Δ A 405 nm/ 30 s (37°C)

Testschema 2-step-CPC:

| 100 µl | Probe |
| 50 µl | Pathromtin SL® |

60 s (37°C)

| 50 µl | 1.7 mM HD-CHG-Ala-Arg-pNA |
| | 6,4 g/l EDTA, 750 mM Arginin, pH 8,7 |

Δ A 405 nm/min (37°C)

Beispiel 3: Optimierung der Substratkonzentration

100 µl normales EDTA-Plasma (gepooltes Normalplasma) mit 250 mM Arginin, pH 8,7, oder normales EDTA-Plasma, welchem 120 mIU/ml Rinder-Thrombin (F IIa) und 30 min danach 250 mM Arginin, pH 8,7, zugesetzt worden waren (= Pathoplasma), sowie Enzymkontrollen mit 12 mIU/ml oder 120 mIU/ml Rinder-Thrombin in 500 mM Arginin, 2 % Haemaccel®, pH 8.7, wurden mit 50 µl HD-CHG-Ala-Arg-pNA in aqua dest. unterschiedlicher Konzentration (0 - 6.67 mM Endkonzentration im Test) für 2 h bei 37°C inkubiert. Vor und nach der Inkubation wurde die Extinktion bei 405 nm bestimmt. Ergebnis: Tabelle 1 zeigt die Enzymaktivitäten von Normalplasma und von Pathoplasma in Abhängigkeit von der finalen Konzentration an chromogenem Substrat HD-CHG-Ala-Arg-pNA.

Tabelle 1: Optimierung der Substratkonzentration

| Finale Substratkonzentr. | Enzymaktiv. Normalplasma | Enzymaktiv. Pathoplasma |
|---|---|---|
| [mM] | [mA/2h] | [mA/2h] |
| 0 | 0 | 0 |
| 0,095 | 43 | 168 |
| 0,19 | 78 | 276 |
| 0,28 | 116 | 412 |
| 0,37 | 149 | 506 |
| 0,41 | 165 | 538 |
| 0,45 | 189 | 580 |
| 0,5 | 210 | 616 |
| 0,55 | 233 | 669 |
| 0,61 | 269 | 707 |
| 0,68 | 304 | 767 |
| 0,76 | 352 | 821 |
| 0,84 | 390 | 868 |
| 0,94 | 401 | 914 |
| 1,04 | 428 | 928 |
| 1,19 | 518 | 999 |
| 1,29 | 623 | 1130 |
| 1,39 | 667 | 1217 |
| 1,58 | 744 | 1268 |
| 1,76 | 839 | 1379 |
| 1,95 | 950 | 1502 |
| 2,18 | 1024 | 1563 |
| 2,38 | 1159 | 1668 |
| 2,67 | 1295 | 1812 |
| 2,97 | 1420 | 1885 |
| 3,33 | 1552 | 2032 |
| 6,67 | > 3000 | > 3000 |

Der Quotient der Extinktionen aus Pathoplasma und Normalplasma nähert sich bei finalen Substrat-Konzentrationen über 1,5 mM dem Wert 1. Je größer dieser Wert, desto spezifischer misst der Test die pathologische Hämostaseaktivierung, hier imitiert durch Zusatz von 120 mIU/ml Rinder-Thrombin. Dieser Quotient erreicht maximale Werte bei Substratkonzentrationen kleiner gleich 1 mM. Jedoch verringert sich die maximal erreichbare Extinktionsänderung bei niedrigen Substratkonzentrationen, insbesondere bei Substratkonzentrationen unter 0,3 mM, d.h. Substratverknappung tritt ein. Daher sollte die finale Substratkonzentration beim GACA zwischen 0,3 mM und 1,0 mM liegen, vorzugsweise zwischen 0,5 mM und 0,8 mM, insbesondere bei 0,56 mM. Bei dieser finalen Substrat-Konzentration wird der Test so spezifisch auf Hämostaseaktivierung, dass gegebenenfalls die Messung der CPC (Ansatz B) unterbleiben kann. Der GACA-Wert entspricht folglich bei einer finalen Substratkonzentration von bis zu 0,8 mM dem uGACA-Wert, d.h. GACA = Ansatz A (ohne Korrektur durch Ansatz B). Nach Zusatz von Thrombin zu EDTA-Plasma wird 90 % des Thrombins inaktiviert, im GACA kann lediglich noch 10 % der zugesetzten Enzymaktivität detektiert werden.

Beispiel 4: <u>Bestimmung des $K_m$-Wertes für Thrombin in Plasma</u>

Normales EDTA-Plasma mit 250 mM Arginin, pH 8,7, und 2. normales EDTA-Plasma, welches mit 120 mIU/ml Rinder-Thrombin und 30 Minuten danach mit 250 mM Arginin, pH 8,7, supplementiert wurde, wurden mit 50 μl HD-CHG-Ala-Arg-pNA in aqua dest. (0 - 6,67 mM final) inkubiert. Die delta A/3h (37°C) wurde gemessen und der bei 1. erhaltene Wert wurde von dem bei 2. erhaltenen Wert abgezogen. Die daraus resultierende Differenz entspricht der Thrombin-Aktivität in Plasma nach Zusatz von 120 mIU/ml Thrombin (F IIa).

Ergebnis: Die Maximalgeschwindigkeit beträgt 800 mA/3h, die Substratkonzentration bei halbmaximaler Geschwindigkeit ($K_m$-Wert) beträgt 0,3 mM.

Beispiel 5: <u>Substraterschöpfung</u>

Um die maximal erreichbaren Extinktionsänderungen und damit die Substraterschöpfung zu erfassen, wurden die Inkubationszeiten von Beispiel 3 auf 24 h verlängert. Extinktionsänderungen größer als 2000 mA werden durch finale Substratkonzentrationen größer als 0,7 mM erreicht. Linearität zwischen Inkubationszeit und Extinktionsänderungen ist bei Einsatz von 0,6 mM Substrat bis zu 1000 mA gewährleistet. Bei Einsatz von Substratkonzentrationen unter 0,4 mM ergeben sich maximale Extinktionsänderungen von unter 1000 mA und eine Linearität zwischen Inkubationszeit und Extinktionsänderung von lediglich unter 500 mA.

Tabelle 2: Substraterschöpfung

| Finale Substratkonzentr. | Maximaler Extinktionsanstieg (405 nm) |
|---|---|
| [mM] | [mA] |
| 0 | 0 |
| 0,095 | 234 |
| 0,19 | 468 |
| 0,28 | 702 |
| 0,37 | 935 |
| 0,41 | 1219 |
| 0,45 | 1435 |
| 0,5 | 1553 |
| 0,55 | 1720 |
| 0,61 | 1853 |
| 0,68 | 2048 |
| 0,76 | 2123 |
| 0,84 | 2184 |
| 0,94 | 2277 |
| 1,04 | 2348 |
| 1,19 | 2523 |
| 1,29 | 2697 |
| 1,39 | 2707 |
| 1,58 | 2714 |
| 1,76 | 2837 |
| 1,95 | 2913 |
| 2,18 | 3050 |
| 2,38 | 3043 |
| 2,67 | 3271 |

(fortgesetzt)

| Finale Substratkonzentr. | Maximaler Extinktionsanstieg (405 nm) |
|---|---|
| [mM] | [mA] |
| 2,97 | 2933 |
| 3,33 | 2827 |
| 6,67 | 2748 |

Beispiel 6: Optimierung der Substratkonzentration bei Arginin/EDTA-Zusatz

Inkubationsbedingungen wie Beispiel 3; allerdings wurde hier als Plasma *STA* Preciclot I® (Roche) unter Zusatz von 3,2 g/l EDTA verwendet. Als Pathoplasma wurde 120 mIU/ml Thrombin- und 250 mM Arginin-supplementiertes EDTA-Plasma STA Preciclot I® verwendet. Das chromogene Substrat HD-CHG-Ala-Arg-pNA befand sich in 750 mM Arginin, 6,4 g/l EDTA, pH 8,7. Die Test-Konzentration an Arginin betrug somit 417 mM. Die Inkubationszeit betrug 3 h (37°C). Ergebnis siehe Tabelle 2. Auch hier ergibt sich eine optimale finale Substratkonzentration bei bis zu 0,8 mM, vorzugsweise 0,05 bis 0,7 mM, insbesondere 0,5 bis 0,6 mM bei diesem GACA-Reaktionsansatz (100 μl Probe + 50 μl Substrat).

Tabelle 3: Optimierung der Substratkonzentration bei 750 mM Arginin-Reagenz

| Finale Substratkonzentr. | Enzymaktiv. Normalplasma | Enzymaktiv. Pathoplasma |
|---|---|---|
| [mM] | [mA/3h] | [mA/3h] |
| 0 | 0 | 0 |
| 0,1 | 33 | 112 |
| 0,14 | 40 | 134 |
| 0,19 | 64 | 213 |
| 0,25 | 74 | 244 |
| 0,33 | 94 | 302 |
| 0,44 | 126 | 391 |
| 0,59 | 160 | 481 |
| 0,79 | 205 | 619 |
| 1,05 | 284 | 731 |
| 1,4 | 372 | 902 |
| 1,87 | 515 | 1094 |
| 2,15 | 721 | 1260 |
| 3,33 | 822 | 1477 |

Beispiel 7: Standardisierung des GACA mit reinem Enzym-Reagenz

Mit 100 μl reinem bovinem Thrombin (0-18 mIU/ml; DadeBehring), reinem bovinem Faktor Xa (0-150 pkat/ml) oder einem Factor-Eight-Inhibitor-Bypassing-Activity-Konzentrat (0-2,6 Einheiten/ml; FEIBA®, Immuno, Wien) in 500 mM Arginin, 2 % Hämaccel, 0,1 % Triton X 100® wurde der GACA (Substrat hier = HD-CHG-Ala-Arg-pNA) durchgeführt. Zusätzliche Proben waren Kontrollplasma N® (KPN) oder mit FEIBA supplementiertes KPN (0-2,6 Einheiten/ml). Die Endinkubation wurde von 0 bis 4 h variiert. Die jeweilige Extinktionsänderung wurde gemessen. Ergebnis: Es zeigt sich ein lineares Verhältnis zwischen eingesetzter Thrombin- oder Faktor Xa-Konzentration und erhaltener Extinktionsänderung, sowie zwischen Inkubationszeit und Extinktionsänderung. 6 mIU/ml IIa oder 45 pkat/ml Xa ergeben eine Extinktionsänderung, die der von normalem Plasma entspricht (100 % GACA). Der GACA kann somit mit reinem Thrombin- oder Faktor Xa- Reagenz kalibriert und standardisiert werden. KPN mit 2,6 Einheiten/ml FEIBA hat einen GACA-Wert von ca. 200 %, d.h. dieser FEIBA-Zusatz zu KPN erhöht den GACA-Wert von 100 % um weitere 100 %. Reines FEIBA im Puffer zeigt die 10 fache Aktivität, d.h. im KPN werden 90 % des zugesetzten FEIBA inaktiviert.

Beispiel 8: Optimum der Reaktionstemperatur und des pH-Wertes beim GACA

GACA wurde durchgeführt mit STA Preciclot I®- Plasma (100 % GACA-Aktivität; Roche), enthaltend zusätzlich 3,2 mg/ml EDTA, mit STA Preciclot I®-Plasma, enthaltend zusätzlich 3,2 mg/ml EDTA und 60 mIU/ml bovinem Thrombin, mit einem Citratplasma-Pool von Patienten, enthaltend zusätzlich 3,2 mg/ml EDTA, mit diesem Citratplasma-Pool, enthaltend zusätzlich 3,2 mg/ml EDTA und 60 mIU/ml Thrombin und mit einem reinen Thrombin-Reagenz enthaltend 6 mIU/ml Thrombin in 500 mM Arginin, 2 % Haemaccel®, 0,1 % Triton X 100®, pH 8,7. Hierfür wurden 100 $\mu$l Probe mit 50 $\mu$l 1,7 mM HD-CHG-Ala-Arg-pNA in 750 mM Arginin, 6,4 g/l EDTA, pH 7,5 bis pH 11 für jeweils 30 min inkubiert. In weiteren Ansätzen wurden sowohl Proben- als auch Reagenzvolumen verdoppelt. Die Inkubationstemperatur betrug 37°C bis 52°C.
Ergebnis: es resultierte ein Aktivitätsmaximum bei 40°C bis 43°C mit etwa der doppelten Extinktionsausbeute verglichen mit 37°C. Daraus folgt, dass durch Anwendung von Reaktions-Temperaturen zwischen 37°C und 49°C, insbesondere zwischen 40°C und 45°C ein höherer Absorptionsanstieg resultiert. Das pH Optimum liegt bei pH 8 bis pH 9. Bei doppeltem Proben- und Reagenzvolumen resultiert aufgrund der Erhöhung der Schichtdicke (d; E = $\varepsilon \cdot c \cdot d$) der Meßküvette die doppelte Extinktionsausbeute (E).

Beispiel 9: Keine Korrelation zwischen Prothrombinzeit, APTT, Fibrinogen und GACA

Bei n=46 Patientenplasmen und KPN (sowohl im GACA als auch im CPC 100 % Aktivität) wurde Prothrombinzeit (PT), APTT, Fibrinogen (Fbgen) bestimmt und der GACA (100 $\mu$l Probe oder 100 $\mu$l 6 mIU/ml Thrombin + 50 $\mu$l 1,7 mM HD-CHG-Ala-Arg-pNA in 6,4 g/l EDTA, 750 mM Arginin, pH 8,7; Bestimmung von delta A / t (hier t = 2 h und Temperatur = 37°C)) durchgeführt. Die Ergebnisse der klassischen Gerinnungsteste PT, APTT und Fibrinogen wurden korreliert mit dem GACA-Wert.
Ergebnis: die Korrelationskoeffizienten (r) betrugen: r (PT/GACA) = 0,062; r (APTT/GACA) = 0,030; r (Fbgen/GACA) = 0,337.
Dies bedeutet, daß der GACA als in-vivo-Gerinnungs-Aktivierungs-Test nicht mit den klassischen Testen der Gerinnung (PT, APTT, Fbgen) korreliert.

Beispiel 10: Probenstabilität

Kontrollplasma N (DadeBehring), versetzt mit 3,2 mg/ml EDTA und Kontrollplasma N, versetzt mit 3,2 mg/ml EDTA und 120 mIU/ml bovinem Thrombin wurden für 0-17 Stunden bei RT gelagert oder bei -80°C eingefroren und bei 37°C aufgetaut und dann der GACA durchgeführt (100 $\mu$l Probe + 50 $\mu$l 1,7 mM HD-CHG-Ala-Arg-pNA in 6,4 g/l EDTA, 750 mM Arginin, pH 8,7).
Ergebnis: Einfrieren / Auftauen bei -80°C / 37°C verändert nicht den GACA-Wert. Die Stabilität von nicht-Arginin-stabilisierten Proben beträgt ca. 2 Stunden. Daraufhin entsteht einerseits in vitro Thrombin-like activity und andererseits verringert sich die Thrombinaktivität in Thrombin-supplementierten Plasmen.

Beispiel 11: Probenstabilisierung durch Arginin

Die Proben aus Beispiel 10 wurden mit 0-300 mM Arginin, pH 8,7 versetzt, für 17 Stunden bei RT gelagert, und dann der GACA (100 $\mu$l Probe + 50 $\mu$l 1,7 mM HD-CHG-Ala-Arg-pNA in 6,4 g/l EDTA, 750 mM Arginin, pH 8,7) durchgeführt.
Ergebnis: Einsatz von mehr als 150 mM Arginin, vorzugsweise mehr als 200 mM Arginin, insbesondere von 250 mM Arginin führt zu einer Stabilisierung der Probe; die Ausgangswerte (GACA-Werte vor 17 stündiger Inkubation) können auch nach 17 Stunden Lagerung reproduziert werden.

Beispiel 12: Optimierung der Aktivierungs-Zeit bei der 2-step-CPC

Der CPC-Test wurde wie folgt durchgeführt: 100 $\mu$l normales EDTA-Plasma wurde mit 50 $\mu$l Pathromtin SL® ($SiO_2$ mit Phospholipiden; DadeBehring) versetzt. Nach einer Aktivierungszeit von 2 Minuten (Raumtemperatur) wurden 50 $\mu$l des GACA-Substrates (hier: 1,7 mM HD-CHG-Ala-Arg-pNA in 750 mM Arginin, 6,4 g/l EDTA, pH 8,7) zugefügt und die Extinktionsänderung / Inkubationszeit (in mA/t) bestimmt.
Ergebnis: Es ergibt sich eine lineare Beziehung zwischen Inkubationszeit und resultierender Extinktionsänderung bei 405 nm (ca. 35 mA/min).
Dann wurde die Aktivierungszeit der 2-step-CPC von 0 bis 7,5 min (RT) variiert. Nach einer Inkubationszeit von 0 bis 16 min (RT) wurde die Extinktionsänderung bei 405 nm bestimmt.

Ergebnis: die optimale Aktivierungszeit bei RT beträgt 1.5 bis 5 min, vorzugs-weise 2 bis 4 min, insbesondere 2.5 - 3 min (entsprechend ca. 1 Minute bei 37°C).

Beispiel 13: <u>Standardisierung des CPC-Testes mit reinem Thrombin-Reagenz</u>

Die 2-step-CPC wurde durchgeführt wie in Beispiel 12 beschrieben, als zusätzliche Probe wurde ein reines Thrombin-Reagenz verwendet (1000 mIU/ml in 500 mM Arginin, 2 % Haemaccel®, 0,1 % Triton X 100®, pH 8,7).

Ergebnis: Die in der Plasmaprobe entstandene Enzymaktivität bei der 2-step-CPC entspricht 165 mIU/ml Thrombin.

Beispiel 14: <u>Inhibition der CPC durch Arginin</u>

Der CPC-Test wurde durchgeführt wie in Beispiel 12 beschrieben, allerdings enthielt das Plasma steigende Mengen (0-300 mM) an Arginin, pH 8,7. Das Substrat war 1,7 mM HD-CHG-Ala-Arg-pNA in aqua dest.

Ergebnis: Ab plasmatischen Arginin-Konzentrationen über 100 mM wird die Pathromtin SL®-CPC auf 0 % (verglichen mit einem Normalplasma ohne Arginin) gedrückt.

Beispiel 15: <u>Optimierung der Substratkonzentration im 1-step-CPC</u>

100 $\mu$l normales EDTA-Plasma und 100 $\mu$l normales EDTA-Plasma, welches mit 120 mIU/ml bovinem Thrombin supplementiert wurde, wurden mit 50 $\mu$l HD-CHG-Ala-Arg-pNA in $H_2O$ ansteigender Konzentration (0 - 1,5 mM Endkonzentration im Test) und 50 $\mu$l Pathromtin SL® für 4 min bei Raum-temperatur inkubiert. Die entstehende Extinktionsänderung wurde in einem Photometer bei 405 nm bestimmt.

Ergebnis: Die maximale Enzymgeschwindigkeit beträgt ca. 650 mA/4min (RT). Halbmaximale Geschwindigkeit wird erreicht bei einer Testkonzentration von ca. 0,4 mM HD-CHG-Ala-Arg-pNA. In diesem Ansatz existiert kein Unterschied zwischen Normalplasma und Pathoplasma, so wie er in Beispiel 3 oder 6 zu sehen ist (s. Tab. 4).

Tabelle 4: Optimierung der Substratkonzentration im 1-Schritt-CPC

| Finale Substratkonzentr. | Enzymaktiv. Normalplasma | Enzymaktiv. Pathoplasma |
|---|---|---|
| [mM] | [mA/4min] | [mA/4min] |
| 0 | 0 | 0 |
| 0,14 | 105 | 123 |
| 0,19 | 180 | 173 |
| 0,27 | 232 | 247 |
| 0,38 | 301 | 342 |
| 0,54 | 454 | 428 |
| 0,76 | 607 | 573 |
| 1,07 | 614 | 526 |
| 1,5 | 595 | 570 |

Beispiel 16: <u>Reaktionskinetik der 1-step-CPC</u>

100 $\mu$l Kontrollplasma N® oder 100 $\mu$l 2000 mIU/ml bovines Thrombin in 500 mM Arginin, 2 % Haemaccel®, 0,1 % Triton X 100®, pH 8,7, wurden inkubiert mit 50 $\mu$l 1,7 mM HD-CHG-Ala-Arg-pNA in $H_2O$ und 50 $\mu$l Pathromtin SL® ($SiO_2$) bei Raumtemperatur (1-step-CPC). Die entstehende Absorption bei 405 nm wurde mittels eines Photometers bestimmt.

Ergebnis: Nach 0, 1, 2, 3, 4, 5, 6, 7, 8, 9, 10 min RT entstanden folgende Extinktionsanstiege zum 0 min Wert:

0 , 610, 940, 1076, 1174, 1234, 1292, 1318, 1334, 1356 mA.

Beispiel 17: <u>Vergleich verschiedener CPC-Ansätze</u>

Bei n=22 Citratplasmen von Patienten und dem Kontrollplasma N® (KPN) wurden folgende CPC-Ansätze durchgeführt: 2-step-Ansätze A, B, C und 1-step-Ansätze D, E, F.

A) 50 $\mu$l Plasma + 25 $\mu$l Pathromtin SL® (SiO$_2$)
B) 50 $\mu$l Plasma + 25 $\mu$l Pathromtin® (Kaolin in H$_2$O zweifach verdünnt)
C) 50 $\mu$l Plasma + 25 $\mu$l Neothromtin® (Ellagsäure)
3 min RT Aktivierungszeit; + 25 $\mu$l 1,7 mM HD-CHG-Ala-Arg-pNA in 750 mM Arginin, 6,4 g/l EDTA, pH 8.7
D) 50 $\mu$l Plasma + 25 $\mu$l 1,7 mM CH$_3$SO$_2$-D-CHG-Gly-Arg-pNA
E) 50 $\mu$l Plasma + 13 $\mu$l 1,5 M Arginin, pH 8,7, + 50 $\mu$l 1,7 mM CH$_3$SO$_2$-D-CHG-Gly-Arg-pNA
F) 50 $\mu$l Plasma + 25 $\mu$l 1,7 mM HD-CHG-Ala-Arg-pNA in H$_2$O + 25 $\mu$l Pathromtin SL®

Bestimmung von delta A bei 405 nm (RT). Die jeweiligen Extinktions-änderungen wurden miteinander korreliert.

Ergebnis: Pathromtin SL®-2-step-CPC mit korrelierte Pathromtin SL®-1-step-CPC mit r = 0,798 (delta A/13 min (RT) für KPN im 2-step-CPC = 354 mA; delta A/2 min (RT) für KPN = 547 mA, d.h. dass bei der 1-step-CPC eine 10-fach höhere Thrombin-Like Activity entsteht verglichen mit der 2-step-CPC; die entsprechenden Werte für Ansatz B) und C) liegen ca. 30 % bzw. 20 % tiefer). Pathromtin-SL®-2-step-CPC korrelierte mit Pathromtin®-2-step-CPC mit r = 0,506, mit Neothromtin®-2-step-CPC mit r = 0,602 und mit CH$_3$SO$_2$-D-CHG-Gly-Arg-pNA-1-step-CPC mit r = 0,502. Eine schwach-negative Korrelation mit r = - 0,533 ergab sich für den Vergleich Pathromtin SL®-1-step-CPC mit CH$_3$SO$_2$-D-CHG-Gly-Arg-pNA-1-step-CPC.

1-step-CPC mit CH$_3$SO$_2$-D-CHG-Gly-Arg-pNA in An- und Abwesenheit von Arginin (hier: 222 mM Arginin final, Ansatz D versus Ansatz E) korrelierten mit r = 0,768. Dies bedeutet, dass die Amidolyse gegenüber CH$_3$SO$_2$-D-CHG-Gly-Arg-pNA selbst in Arginin-stabilisiertem Plasma sicher bestimmt werden kann, was für chromogene Substrate mit CH$_3$SO$_2$-Gruppe gilt.

Beispiel 18: <u>Erhöhung der Extinktion (E) durch Vergrößerung der Schichtdicke (d; E = ε·c·d)</u>

Die Eigenextinktionen von n= 290 Citratplasmen von Patienten wurden in einer Mikrotiter-platte (100 $\mu$l Probenvolumen) gemessen.

Ergebnis: Die mittlere Eigenextinktion bei 405 nm betrug 210,1 mA, die Standardabweichung war 72,3 mA. Dies bedeutet, dass bei Verwendung einer 4-8 fachen Schichtdicke (d) die notwendige Reaktionszeit, um ca. 300 mA Extinktionsdifferenz zu erzielen, auf 25 % bzw. auf 12.5 % verkürzt werden kann, was einem Extinktionsanstieg von 300 mA/ 30 bzw. 15 min (bei 37°C) entspricht. Bei Verwendung eines entsprechend sensitiven Photometers beträgt somit die GACA-Aktivität eines Normalplasmas 10-30 mA/min. Plasmen mit hoher Eigenextinktion (> 200 % der Norm) werden mit 500 mM Arginin verdünnt.

Beispiel 19: <u>VK-Werte GACA und CPC</u>

GACA (100 $\mu$l Probe + 50 $\mu$l HD-CHG-Ala-Arg-pNA in 6,4 g/l EDTA, 750 mM Arginin, pH 8,7) und CPC (100 $\mu$l Probe + 50 $\mu$l 1,7 mM HD-CHG-Ala-Arg-pNA, 6.4 g/l EDTA + 50 $\mu$l Pathromtin SL®) wurden mit Kontrollplasma N®, STA Preciclot I®, STA Preciclot II® und mit gepooltem Patientenplasma in 10fach-Bestimmung durchgeführt.

Ergebnis: Die intra-assay-VK Werte für GACA und CPC liegen zwischen 1 % und 4 %.

Beispiel 20: <u>Nachweis von akivem Trypsin in Plasma</u>

25 $\mu$l Pankreatitis-Patientenplasma (Citrat) , 25 $\mu$l KPN, 25 $\mu$l 36 pkat/ml boviner Faktor Xa in PBS, 0.1 % Triton X 100®, wurden mit 25 $\mu$l Testpuffer, enthaltend 600 mM NaHCO$_3$, pH 8.7 mit und ohne 600 mM Arginin, 25 $\mu$l 25 mM Chloramin T®, 25 $\mu$l 3 mM N-a-Z-D-Arg-Gly-Arg-pNA.2HCl (S-2765®; Chromogenix, Mölndal, Schweden) und 25 $\mu$l Aprotinin (0, 2, 20, 200, 2000, 20000 KIU/ml) für 50 min bei 37°C inkubiert und der Anstieg der Extinktion bei 405 nm während dieser Inkubationszeit mittels eines Mikrotiterplatten-Photometers detektiert.

Ergebnis: Der Einsatz von 25 $\mu$l 0, 2, 20, 200, 2000, 20000 KIU/ml resultierte in Anwesenheit von Arginin Extinktionsanstiege von 788, 778, 709, 685, 497, 259 mA, beziehungsweise, und in Abwesenheit von Arginin Extinktionsanstiege von 738, 733, 715, 531, 294, 103 mA. Die diesbezüglichen Extinktionsanstiege im KPN waren 131, 133, 126, 126, 125, 123 mA in Anwesenheit von Arginin und 122, 134, 135, 108, 118, 124 mA in Abwesenheit von Arginin. Reiner Faktor Xa - im Gegensatz zu aktivem Trypsin (wie andere aktive Serinproteasen interagierend mit α2-Macroglobulin) - wird nicht von Aprotinin inhibiert. Mit dem erfindungsgemäßen Testsystem kann aktives Trypsin in Blut nachgewiesen werden, ein Marker für das Vorliegen einer Pankreatitis und/oder für den Schweregrad einer solchen.

Verwendete Abkürzungen

| A | Absorptionseinheiten |
|---|---|
| Abu | L-alpha-Aminobuttersäure |
| Ala | Alanin |
| APTT | aktivierte partielle Thromboplastinzeit |
| Arg | Arginin |
| But | Butyl |
| Bz | Benzoyl |
| CHA | ß-Cyclohexylalanin |
| $CH_3CO$ | Acetyl |
| $CH_3OCO$ | Methoxycarbonyl |
| $CH_3SO_2$ | Methylsufonyl |
| CHG | Cyclohexylglycin |
| CPC | Contact Phase Capacity |
| d | Schichtdicke |
| DIC | Disseminated Intravascular Coagulation |
| E | Extinktion |
| EDTA | Ethylene Diamine Tetraacetic acid |
| EGTA | Ethylene Glycol-bis(ß-aminoethyl Ether)N,N,N',N'-Tetraacetic Acid |
| Enzy.-akt. | Enzymaktivität |
| F IIa | Faktor IIa, Thrombin |
| Fbgen | Fibrinogen |
| FEIBA | Factor-Eight-Inhibitor-Bypassing-Activity |
| GACA | Global Assay of in-vivo Coagulation Activation |
| Glu | Glutaminsäure |
| Gly | Glycin |
| HHT | Cyclohexyltyrosin |
| Ile | Isoleucin |
| KIU | Kallikrein Inhibiting Units |
| $K_m$ | Michaelis-Konstante |
| KPN | Kontrollplasma N® |
| Leu | Leucin |
| Lys | Lysin |
| mA | milli Absorptionseinheiten |
| mIU | milli International Units |
| mM | mmol/l |
| PBS | Phosphate Buffered Saline |
| Phe | Phenylalanin |
| Pip | Pipecolinsäure |
| Pro | Prolin |
| PT | Prothrombinzeit |
| pNA | para-Nitroanilid |
| Suc | Succinyl |
| t | Zeit |
| TT | Thrombinzeit |
| uGACA | unkorrigierte GACA |
| Val | Valin |
| VK | Variationskoeffizient |

**Patentansprüche**

1. Verfahren zur Bestimmung der in-vivo Aktivität von Proteasen der Hämostase oder von Trypsin oder Subtilisin einer biologischen Flüssigkeit, **dadurch gekennzeichnet, dass** die biologische Flüssigkeit mit mindestens einem chromogenen oder fluorogenen Substrat für mindestens ein Hämostaseenzym oder für Trypsin und/oder für Subtilisin inkubiert wird und vor oder mit Zugabe des chromogenen oder fluorogenen Substrates mindestens ein Antikoagulans aus der Gruppe EDTA, EGTA, Arginin, Guanidinium und Singlet Oxygen Erzeuger hinzugegeben wird.

2.  Verfahren zur Bestimmung der korrigierte Koagulationsaktivierung GACA, **dadurch gekennzeichnet, dass** die unkorrigierte Koagulationsaktivierung uGACA durch das Verfahren nach Anspruch 1 bestimmt wird und der erhaltene Wert um die Kontaktphasenkapazität CPC korrigiert wird .

3.  Verfahren nach einem der vorhergehenden Ansprüche, **dadurch gekennzeichnet, dass** es sich bei der biologischen Flüssigkeit um eine Kompartmentflüssigkeit, um Blut und/oder um Plasma handelt.

4.  Verfahren nach Anspruch 3, **dadurch gekennzeichnet, dass** es sich bei dem Blut oder Plasma um EDTA-Blut, EDTA-Plasma, EDTA/Arginin-Blut oder EDTA/Arginin-Plasma, EDTA/Guanidin-Blut oder EDTA/Guanidin-Plasma, EDTA/Chloramin T-Blut oder EDTA/Chloramin T-Plasma handelt.

5.  Verfahren nach einem der vorangehenden Ansprüche, **dadurch gekennzeichnet, dass** das chromogene oder fluorogene Substrat in einer Konzentration von 0,05 bis 1 mM eingesetzt wird und/oder in einer Menge von bis zu 3 nmol/μl Probe.

6.  Verfahren nach einem der vorangehenden Ansprüche, **dadurch gekennzeichnet, dass** das chromogene oder fluorogene Substrat in einer Konzentration von 0,5 bis 0,6 mM eingesetzt wird und/oder in einer Menge von bis zu 1 ,5 nmol/μl Probe.

7.  Verfahren nach Anspruch 1 umfassend folgende Schritte:

    a) zu der zu untersuchenden Probe und zu einer Standardprobe jeweils enthaltend eine biologische Flüssigkeit wird mindestens ein chromogenes und/oder fluorogenes Substrat für mindestens ein Hämostaseenzym oder für Trypsin und/oder für Subtilisin hinzugegeben.
    b) die Absorption, Fluoreszenz und/oder Extinktion der so erhaltenen Probe bzw. Standardprobe wird bei mindestens einer definierten Wellenlänge bestimmt,
    c) es erfolgt Inkubation der Probe bzw. Standardprobe,
    d) die Absorption, Fluoreszenz und/oderExtinktion der Probe bzw. Standardprobe bei der definierten Wellenlänge wird erneut bestimmt oder die Änderung der Absorption, Fluoreszenz und/oder Extinktion der Probe während der Inkubation der Probe bzw. Standardprobe wird verfolgt,
    e) die Hämostaseaktivierung oder die Aktivität von Trypsin und/oder von Subtilisin wird als Differenz der Extinktionswerte aus (b) und (d) ermittelt.

8.  Verfahren nach Anspruch 1 umfassend folgende Schritte:

    a) zu der zu untersuchenden Probe und der Standardprobe jeweils enthaltend eine biologische Flüssigkeit wird mindestens ein chromogenes und/oder fluorogenes Substrat für mindestens ein Hämostaseenzym zusammen mit mindestens einem Kontaktphasenaktivator hinzugegeben,
    b) die Absorption, Fluoreszenz und/oder Extinktion der so erhaltenen Probe bzw. Standardprobe wird bei mindestens einer definierten Wellenlänge bestimmt,
    c) es erfolgt Inkubation der Probe bzw. Standardprobe,
    d) die Absorption, Fluoreszenz und/oder Extinktion der Probe bzw. Standardprobe bei der definierten Wellenlänge wird erneut bestimmt oder die Änderung der Absorption, Fluoreszenz und/oder Extinktion der Probe während der Inkubation der Probe bzw. Standardprobe wird verfolgt,
    e) die Hämostaseaktivierung wird als Differenz der Extinktionswerte aus (b) und

9.  Verfahren nach einem der vorhergehenden Ansprüche, **dadurch gekennzeichnet, dass** das chromogene und/oder fluorogene Substrat eine durch mindestens ein Hämostaseenzym spaltbare Aminosäuresequenz umfasst.

10. Verfahren nach einem der vorhergehenden Ansprüche, **dadurch** gekenntzeichnet, dass das chromogene und/oder fluorogene Substrat eine durch mindestens eine durch Faktor Xa oder Thrombin spaltbare Aminosäuresequenz umfasst.

11. Verfahren nach Anspruch 9, **dadurch gekennzeichnet, dass** es sich bei dem chromogenen Substrat um eine Verbindung handelt, die ausgewählt wird aus der Gruppe bestehend aus HD-CHG-Ala-Arg-pNA. 2AcOH; HD-CHA-Ala-Arg-pNA; HD-Ile-Phe-Pip-Arg-pNA · HCl; HD-HHT-Gly-Arg-pNA; N-$\alpha$-Z-D-Arg-Gly-Arg-pNA·2HCl; Bz-Ile-Glu($\gamma$-OR)Gly-Arg-pNA·HCl; HD-Ile-Pro-Arg-pNA · HCl; -CO-Ile-Glu-($\gamma$-OR)-Gly-Arg-pNA; HD-Val-Leu-Lys-Arg-pNA und D-Pro-Phe-Arg-pNA.

12. Verfahren nach einem der vorhergehenden Ansprüche, **dadurch gekennzeichnet, dass** der biologischen Flüssigkeit vor oder mit Zugabe des chromogenen oder fluorogenen Substrates ein Singlet Oxygen Erzeuger aus der Gruppe der Chloramine hinzugegeben wird.

## Claims

1. Procedure to determine the in-vivo activity of proteases of hemostasis or of trypsin or of subtilisin in a biologial fluid, **characterized** thereby that the biological fluid is incubated with at least one chromogenic or fluorogenic substrate for at least one protease of hemostasis or for trypsin and/or for subtilisin and prior to or together with the addition of the chromogenic or fluorogenic substrate at least one anticoagulant from the group EDTA, EGTA, arginine, guanidine and generator of singlet oxygen is added.

2. Procedure to determine the corrected activation of coagulation GACA, **characterized** thereby that the uncorrected activation of coagulation uGACA is determined by means of the procedure according to claim 1 and the obtained value is corrected by the capacity of the phase of contact.

3. Procedure according to one of the above claims, **characterized** thereby that the biological fluid is a fluid of compartment, blood and/or plasma.

4. Procedure according to claim 3, **characterized** thereby that the blood or plasma is EDTA-blood, EDTA-plasma, EDTA/arginine-blood or EDTA/arginine-plasma EDTA/guanidine-blood or EDTA/guanidine-plasma, EDTA/Chloramine T-blood or EDTA/Chloramine T-plasma.

5. Procedure according to one of the above claims, **characterized** thereby that the chromogenic or fluorogenic substrate is used in a concentration of 0.05 to 1 mM and/or in an amount of up to 3 nmoles/$\mu$l of sample.

6. Procedure according to one of the above claims, **characterized** thereby that the chromogenic or fluorogenic substrate is added in a concentration of 0.5 to 0.6 mM and/or in an amount of up to 1.5 nmol/$\mu$l of sample.

7. Procedure according to claim 1, that comprises the following steps:

   a) to the sample that shall be analyzed or to the standard-sample that contains a biologic fluid at least one chromogenic and/or fluorogenic substrate for at least one enzyme of hemostasis or for trypsin and/or subtilisin is added,
   b) the absorption, fluorescence, and/or extinction of the so-obtained sample or standard-sample is determined at at least one defined length of wave,
   c) the sample or the standard-sample are incubated,
   d) the absorption, fluorescence, and/or extinction of the sample or the standard-sample is determined again at a defined length of wave or the change of absorbance, of fluorescence and/or of extinction of the sample is measured during the incubation of the sample or the standard-sample,
   e) the activation of hemostasis or the activity of trypsin and/or of subtilisin is determined as the difference of the values of extinction from (b) and (d).

8. Procedure according to claim 1 that comprises the following steps:

   a) to the sample that shall be analyzed or to the standard-sample that contains a biologic fluid at least one chromogenic and/or fluorogenic substrate for at least one enzyme of hemostasis is added together with at least one activator of the contact-phase,
   b) the absorption, fluorescence, and/or extinction of the so-obtained sample or standard-sample is determined at at least one defined length of wave,
   c) the sample or the standard-sample are incubated,
   d) the absorption, fluorescence, and/or extinction of the sample or the standard-sample is determined again at a defined length of wave or the change of absorbance, of fluorescence and/or of extinction of the sample is measured during the incubation of the sample or the standard-sample,
e) the activation of hemostasis or the activity of trypsin and/or of subtilisin is determined as the difference of the values of extinction from (b) and (d).

9. Procedure according to one of the above claims, **characterized** thereby that the chromogenic and/or fluorogenic substrate contains a sequence of amino acids that can be degraded by at least one enzyme of hemostasis.

10. Procedure according to one of the above claims, **characterized** thereby that the chromogenic and/or fluorogenic substrate contains a sequence of amino acids that can be degraded by factor Xa or by thrombin.

11. A method according to claim 9, **characterized** thereby that the chromogenic substrate is a compound from a group that consist of
HD-CHG-Ala-Arg-pNA.2AcOH; HD-CHA-Ala-Arg-pNA;
HD-Ile-Phe-Pip-Arg-pNA.HCl; HD-HHT-Gly-Arg-pNA;
N-alpha-Z-D-Arg-Gly-Arg-pNA.2HCl; HD-Ile-Pro-Arg-pNA.HCl;
-CO-Ile-Glu-(gamma-OR)-Gly-Arg-pNA; HD-Val-Leu-Arg-pNA;
and D-Pro-Phe-Arg-pNA.

12. Procedure according to one of the above claims, **characterized** thereby that to the biologic fluid prior to or together with the addition of the chromogenic or fluorogenic substrate a generator of singlet oxygen from the group of the chloramines is added.

## Revendications

1. Procédure à déterminer l'in vivo l'activité de protéases de l'hémostase ou de trypsine ou de subtilisine dans un fluide biologique, **caractérisé par** là que le liquide biologique est incubé avec au moins un substrat chromogénique ou fluorogenique pour au moins un de protéase de l'hémostase ou de trypsine et/ou de subtilisine et avant ou en même temps avec l'ajout du substrat chromogène ou fluorogenique au moins un anticoagulant du groupe EDTA, EGTA, arginine, guanidine et générateur d'oxygène singulet est ajouté.

2. Procédure à déterminer la corrigé activation de la coagulation GACA, **caractérisée** ainsi que la activation sans correction de la coagulation uGACA est déterminé par le biais de la procédure selon la revendication 1 et la valeur obtenue est corrigée par la capacité de la phase de contact.

3. Procédure selon une de ces revendications, ainsi que l' fluide biologique est un compartiment de liquide, sang et / ou plasma.

4. Procédure selon la revendication 3, **caractérisée** ainsi que le sang ou le plasma est EDTA-sang, EDTA-plasma, EDTA / arginine-sang ou EDTA / arginine-plasma, EDTA / guanidine- sang ou de EDTA / guanidine-plasma, EDTA / chloramine T - sang ou EDTA /chloramine T-plasma.

5. Procédure selon une de ces revendications, ainsi que le substrat chromogénique ou fluorogenique est utilisé à une concentration de 0,05 à 1 mM et / ou dans un montant maximum de 3 nmoles / $\mu$l de l'échantillon.

6. Procédure selon une de ces revendications, ainsi que le substrat chromogénique ou fluorogenique est ajouté à une concentration de 0,5 à 0,6 mM et / ou dans un montant maximum de 1,5 nmol / $\mu$l de l'échantillon.

7. Procédure selon la revendication 1, qui comprend les étapes suivantes:

   a) à l'échantillon qui doit être analysé ou à l'standard-échantillon qui contient un liquide biologique au moins un substrat chromogène et / ou fluorogenique pendant au moins une enzyme de l'hémostase ou de trypsine et / ou de subtilisine est ajouté,
   b) l'absorption, la fluorescence, et / ou l'extinction de ce que l'on a obtenu des échantillons ou de l'standard-échantillon est déterminé à au moins une durée définie de la vague,
   c) l'échantillon ou l'standard-échantillon sont incubés,
   d) l'absorption, la fluorescence, et / ou l'extinction de l'échantillon ou le standard-échantillon est déterminé à nouveau à une longueur définie de l'onde ou le changement d'absorption, de fluorescence et / ou d'extinction de l'échantillon est mesuré au cours de l'incubation de l'échantillon ou de l'standard-échantillon,

e) l'activation de l'hémostase ou l'activité de trypsine et / ou de subtilisine est déterminée comme étant la différence des valeurs de l'extinction de (b) et (d).

8. Procédure selon la revendication 1, qui comprend les étapes suivantes:

a) à l'échantillon qui doit être analysé ou à l'standard-échantillon qui contient un liquide biologique au moins un substrat chromogène et / ou fluorogenique pendant au moins une enzyme de l'hémostase est ajouté avec au moins un activateur de la phase de contact,

b) l'absorption, la fluorescence, et / ou l'extinction de ce que l'on a obtenu des échantillons ou de l'standard-échantillon est déterminé à au moins une durée définie de la vague,

c) l'échantillon ou l'standard-échantillon sont incubés,

d) l'absorption, la fluorescence, et / ou l'extinction de l'échantillon ou le standard-échantillon est déterminé à nouveau à une longueur définie de l'onde ou le changement d'absorption, de fluorescence et / ou d'extinction de l'échantillon est mesuré au cours de l'incubation de l'échantillon ou de l'standard-échantillon,

e) l'activation de l'hémostase ou l'activité de trypsine et / ou de subtilisine est déterminée comme étant la différence des valeurs de l'extinction de (b) et (d).

9. Procédure selon une de ces revendications, ainsi que le substrat chromogénique et / ou fluorogenique contient une séquence d'acides aminés qui peuvent être dégradés par au moins une enzyme de l'hémostase.

10. Procédure selon une de ces revendications, ainsi que le substrat chromogénique et / ou fluorogenique contient une séquence d'acides aminés qui peuvent être dégradés par facteur Xa ou par thrombine.

11. Une méthode selon la revendication 9, **caractérisée** ainsi que le substrat chromogénique est un composé d'un groupe qui se composent de
HD-CHG-Ala-Arg-pNA.2AcOH; HD-CHA-Ala-Arg-pNA; HD-Ile-Phe-Pip-Arg-pNA.HCl;
HD-HHT-Gly-Arg-pNA; N-alpha-Z-D-Arg-Gly-Arg-pNA.2HCl;
HD-Ile-Pro-Arg-pNA.HCl; -CO-Ile-Glu-(gamma-OR)-Gly-Arg-pNA;
HD-Val-Leu-Arg-pNA et D-Pro-Phe-Arg-pNA.

12. Procédure selon une de ces revendications, ainsi que pour les fluides biologiques avant ou en même temps avec l'ajout du chromogène ou fluorogenic substrat un générateur d'oxygène singulet du groupe des chloramines est ajouté.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19904674 A
- DE 19940389 A
- DE 19756773 A

**In der Beschreibung aufgeführte Nicht-Patentliteratur**

- **KHER et al.** *Haemostasis,* 1997, vol. 27 (5), 211-8
- **STIEF.** *Thromb Haemost,* 2000, vol. 84, 1120-1
- **MACCALLUM et al.** *Thromb Haemost,* 2000, vol. 83 (3), 421-6
- **BOS et al.** *Thromb Haemost,* 1999, vol. 81 (3), 467-8
- **CARDIGAN et al.** *Blood Coagul Fibrinolysis,* 1998, vol. 9 (4), 323-32
- *Thrombosis Research,* 2000, vol. 97 (4), 231-7
- **KYLÄNPÄÄ-BÄCK ML et al.** *JOP. Journal of the Pancreas (Online,* 2002, vol. 3, 34-48